# EUROPEAN PATENT APPLICATION

(11) **EP 4 368 611 A1**
(43) Date of publication of application: **15.05.2024**
(21) Application number: 21949451.5
(22) Date of filing: 23.12.2021
(51) Int. Cl.: C07D 213/46, A61K 31/44, A61K 31/404, A61K 31/4439, A61P 29/00, C07D 209/12, C07D 401/06

(54) **NOVEL COMPOUND EXHIBITING THERAPEUTIC EFFECT ON INFLAMMATORY DISEASE AS P38 MAP KINASE INHIBITOR**

(30) Priority: 06.07.2021 KR 20210088499
(71) Applicant: Prazertherapeutics Inc., Seoul 02455 (KR)
(72) Inventor: INN, Kyung-Soo, Goyang-si Gyeonggi-do 10491 (KR); KIM, Nam-Jung, Seoul 06356 (KR); LEE, Jong Kil, Seoul 02567 (KR); KIM, Ga Yeong, Seoul 05837 (KR); KIM, Kyeojin, Seoul 06356 (KR); KIM, Donghwan, Seoul 02811 (KR); DO, Jimin, Seoul 05354 (KR); SONG, Chaewon, Seoul 05245 (KR); LEE, Na-Rae, Namyangju-si Gyeonggi-do 12113 (KR)
(74) Representative: Roos, Peter
(86) International application number: PCT/KR2021/019687
(87) International publication number: WO 2023/282414

(57) **Abstract**

The present invention relates to a novel compound exhibiting anti-inflammatory activity, and the compound of the present invention plays a critical role in the generation of pro-inflammatory cytokines, thereby having excellent inhibitory activity against p38 MAPK, which is known to cause inflammatory diseases, and thus can be effectively used as an agent for treating inflammatory diseases.

## Description

### Technical Field

The present disclosure relates to a novel compound exhibiting an anti-inflammatory activity, wherein the novel compound of the present disclosure has an excellent p38 MAP kinase inhibitory efficacy and thus is useful as a therapeutic agent for inflammatory diseases.

### Background Art

p38 is one of serine/threonine kinases belonging to a mitogen-activated protein kinase (MAPK) family. A MAPK pathway is activated by various extracellular stimuli and converts to cause various intracellular reactions through phosphorylation of specific substrates in the cell, subtypes of which include JNK and ERK in addition to p38. MAPK is a protein kinase that is activated by insulin, mitogen, growth factors, and stimulation by activation of immune cells, and plays an important role in a signaling pathway to transmit signals from a receptor on the cell surface into the cell.

The p38 signaling system regulates processes such as metabolism and apoptosis, and undergoes a series of phosphorylation processes in which a kinase at an upper level transfers a phosphate group to a kinase at a lower level. p38-α, p38-β, p38-γ, and p38-δ have been discovered as p38 MAPK which are activated by osmotic shocks, inflammatory cytokines, lipopolysaccharide (LPS), ultraviolet (UV), growth factors (GFs), and the like.

Although inflammation is known to occur by a variety of causes, studies have shown that MAPK may be activated by a variety of stressful stimuli, and especially p38 MAPK plays a crucial role in production of pro-inflammatory cytokines such as TNF-α, IL-1β, and IL-6 to induce inflammatory diseases (NATURE REVIEWS, Drug discovery (2003), Vol.2, 717-726). Thereby, several small molecule compounds have been developed as an inhibitor of p38 MAPK (as in Korean Patent No. 10-1879481), and several small molecule inhibitors of p38 act as an inflammatory regulator by inhibiting IL-1 and TNF synthesis in human monocytes at a low concentration, such that inhibition of these cytokines has been revealed to be able to regulate, mitigate, or alleviate inflammatory responses.

In this regard, the inventors of the present disclosure have completed the present disclosure as a result of researching a novel compound having an anti-inflammatory activity through p38 MAPK inhibition.

### Disclosure of the Invention

### Technical Goals

An object of the present disclosure is to provide a novel compound having an excellent anti-inflammatory efficacy by effectively inhibiting p38 MAP kinase for effective treatment of an inflammatory disease.

### Technical Solutions

To achieve the above objective, the present disclosure provides a compound represented by the following Chemical Formula 1 or a pharmaceutically acceptable salt thereof.

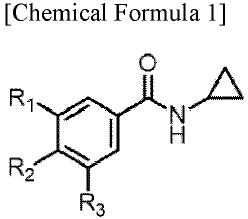

In the Chemical Formula,
R₁ is 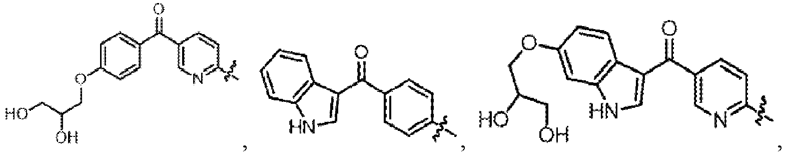 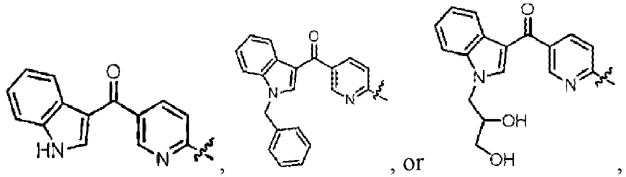
R₂ is H, halogen, or -C1-C6, and
R₃ is H or halogen.

Specifically, the compound of the present disclosure may include compounds in Table 1 below or pharmaceutically acceptable salts thereof.

**TABLE 1**

| Compound | Chemical Name | Structural Formula |
|---|---|---|
| 1 | N-Cyclopropyl-3-(5-(4-(2,3-dihydroxypropoxy)benzoyl)pyr idin-2-yl)-5-fluoro-4-methylbenzamide | 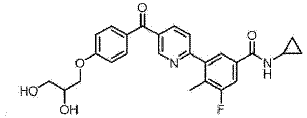 |
| 2 | 6-Chloro-N-cyclopropyl-4'-(1H-indole-3-carbonyl)-[1,1'-biphenyl]-3-carboxamide | 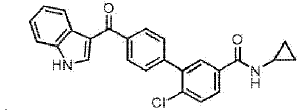 |
| 3 | N-Cyclopropyl-3-(5-(6-(2,3-dihydroxypropoxy)-1H-indole-3-carbonyl)pyridin-2-yl)-5-fluoro-4-methylbenzamide | 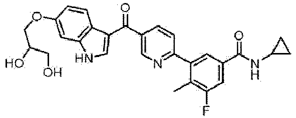 |
| 4 | 3-(5-(1H-indole-3-carbonyl)pyridin-2-yl)-N-cyclopropyl-5-fluoro-4-methylbenzamide | 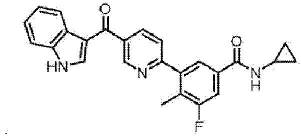 |
| 5 | 3-(5-(1-Benzyl-1H-indole-3-carbonyl)pyridin-2-yl)-N-cyclopropyl-5-fluoro-4-methylbenzamide | 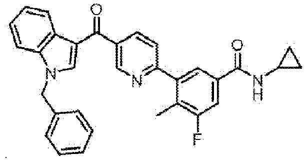 |
| 6 | N-Cyclopropyl-3-(5-(1-(2,3-dihydroxypropyl)-1H-indole-3-carbonyl)pyridin-2-yl)-5-fluoro-4-methylbenzamide | 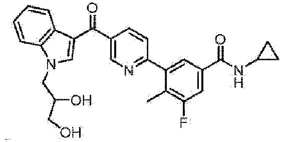 |

It is possible to provide a composition for treating or preventing an inflammatory disease, including a compound according to the present disclosure.

### Advantageous Effects

Since a compound of the present disclosure has an excellent inhibitory efficacy against p38 MAPK, which is known to plays a decisive role in production of pro-inflammatory cytokines and cause an inflammatory disease, the compound may be useful as a therapeutic agent for the inflammatory disease.

### Brief Description of Drawings

FIGS. 1 and 2 show relative mRNA expression levels of the inflammatory cytokines IL-1β and TNFα of compounds according to the present disclosure.
FIGS. 3 to 5 show relative mRNA expression levels of the inflammatory cytokines IL-1β, TNFα, and IL-6 of compounds according to the present disclosure.

### Best Mode for Carrying Out the Invention

Hereinafter, aspects and example embodiments of the present disclosure will be described in detail so that ordinary skilled person in the art to which the present disclosure pertains may easily implement in reference to the accompanied drawings. However, the present disclosure may be implemented in various forms and is not limited to aspects and example embodiments described herein.

Throughout the specification of the present application, when a part "comprises" or "includes" a component, this means that the part may further include other components rather than excluding other components, unless specifically stated to the contrary.

The term "pharmaceutically acceptable salt" as used herein refers to a salt that is pharmaceutically acceptable and maintains a desired pharmacological activity of its parent compound. The salts are not particularly limited if they are pharmaceutically acceptable.

As used herein, the term "alkyl" refers to a hydrocarbon having a primary, secondary, tertiary, or quaternary carbon atom, and includes a saturated aliphatic group that may be straight chained, branched, or cyclic, or a combination thereof. For example, an alkyl group may have 1 to 20 carbon atoms (i.e., C₁-C₂₀ alkyl), 1 to 10 carbon atoms (i.e., C₁-C₁₀ alkyl), or 1 to 6 carbon atoms (i.e., C₁-C₆ alkyl). Examples of suitable alkyl groups may include, but is not limited to, methyl (Me, -CH₃), ethyl (Et, -CH₂CH₃), 1-propyl (n-Pr, n-propyl, -CH₂CH₂CH₃), 2-propyl (i-Pr, i-propyl, -CH(CH₃)₂), 1-butyl (n-Bu, n-butyl, -CH₂CH₂CH₂CH₃), 2-methyl-1-propyl (i-Bu, i-butyl, -CH₂CH(CH₃)₂), 2-butyl (s-Bu, s-butyl, -CH(CH₃)CH₂CH₃), 2-methyl-2-propyl (t-Bu, t-butyl, -C(CH₃)₃), 1-pentyl (n-pentyl, -CH₂CH₂CH₂CH₂CH₃), 2-pentyl (-CH(CH₃)CH₂CH₂CH₃), 3-pentyl (-CH(CH₂CH₃)₂), 2-methyl-2-butyl (-C(CH₃)₂CH₂CH₃), 3-methyl-2-butyl (-CH(CH₃)CH(CH₃)₂), 3-methyl-1-butyl (-CH₂CH₂CH(CH₃)₂), 2-methyl-1-butyl (-CH₂CH(CH₃)CH₂CH₃), 1-hexyl (-CH₂CH₂CH₂CH₂CH₂CH₃), 2-hexyl (-CH(CH₃)CH₂CH₂CH₂CH₃), 3-hexyl (-CH(CH₂CH₃)(CH₂CH₂CH₃)), 2-methyl-2-pentyl (-C(CH₃)₂CH₂CH₂CH₃), 3-methyl-2-pentyl (-CH(CH₃)CH(CH₃)CH₂CH₃), 4-methyl-2-pentyl (-CH(CH₃)CH₂CH(CH₃)₂), 3-methyl-3-pentyl (-C(CH₃)(CH₂CH₃)₂), 2-methyl-3-pentyl (-CH(CH₂CH₃)CH(CH₃)₂), 2,3-dimethyl-2-butyl (-C(CH₃)₂CH(CH₃)₂), 3,3-dimethyl-2-butyl (-CH(CH₃)C(CH₃)₃), and octyl (-(CH₂)₇CH₃).

Furthermore, the term "alkyl" as used throughout the specification, example embodiments, and claims is intended to encompass all unsubstituted and substituted alkyl groups.

The terms "halo" and "halogen" as used herein refer to halogens and include chloro, fluoro, bromo, and iodo.

The term "Cx-y" or "Cx-Cy", when used in conjunction with chemical residues such as alkyl, alkenyl or alkynyl, is considered to include a group having x to y carbons within its chain. C₀ alkyl represents hydrogen when the group is at its terminal position and bonds when it is inside. For example, the C₁-C₂₀ alkyl group includes 1 to 20 carbon atoms in its chains.

Compounds according to the present disclosure may be prepared from readily available starting materials using modifications for a synthesis protocol described below that is well known to those skilled in the art.

### Modes for Carrying Out the Invention

Hereinafter, the present disclosure will be described in more detail through example embodiments, but the following example embodiments are for illustrative purposes only and are not intended to limit the scope of the present disclosure.

### [Preparation Example]

Instruments and reagents used in methods of synthesizing compounds according to the present disclosure

All starting materials and reagents used in the synthesis of the compounds of the present disclosure were purchased from Aldrich, Alfa Aesar, TCI and used without any purification process. Glassware used in a reaction was in a state of being dried in an oven at 80°C, and argon gas or nitrogen gas was injected to block the air and moisture, followed by the reaction.

For flash column chromatography of the present disclosure, silica gel 60 (230-400 mesh) was used by appropriately adjusting solvents such as hexane, ethyl acetate, dichloromethane, and methanol. A 0.25 mm silica gel plate was used for thin-layer chromatography for analysis.

The ¹H-NMR spectra and ¹³C-NMR spectra of the present disclosure were analyzed using a Bruker Avance 400 (400 MHz for 1H; 100 MHz for 13C) spectrometer or a VARIAN VNMRS 500 (500 MHz for 13C) spectrometer. ¹H and ¹³C NMR chemical shift values were expressed in ppm using tetramethylsilane (TMS) as an internal standard. NMR signals were represented as m (multiplet), s (singlet), d (doublet), t (triplet), q (quartet), quin (quintet), bs (broad singlet), bd (broad doublet), dd (doublet of doublets), dt (doublet of triplets), and dq (doublet of quartets), and the binding constant was expressed in hertz (Hz).

The low resolution mass spectra and high resolution mass spectra of the present disclosure were measured using the VG Trio-2 GC-MS or JEOL JMS-700 instruments.

In one aspect, intermediate Compound 10 of the present disclosure may be prepared according to the following scheme 1.

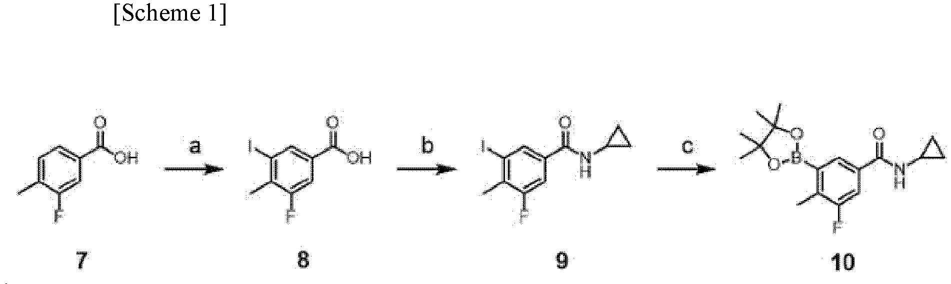

Reagents and conditions: (a) N-iodosuccinimide, sulfuric acid, H₂O, r.t., 85%; (b) cyclopropylamine, ethyl-3-(3-dimethylaminopropyl)carbodiimide, N,N-diisopropylethylamine, 1-hydroxybenzotriazole hydrate, DMF, r.t, 82%; (c) bis(pinacolato)diboron, PdCl₂(dppf)₂, KOAc, DMF, 90°C, 50%.

Compound 8 was synthesized through iodination reaction with commercially available 3-fluoro-4-methylbenzoic acid (Compound 7) as the starting material, and then Compound 9 was synthesized through EDC coupling with cyclopropylamine. Major intermediate Compound 10 was synthesized through cross-coupling with bis(pinacolato)diboron.

In one aspect, intermediate Compounds 16 to 21 of the present disclosure may be prepared according to the following Scheme 2.

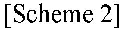

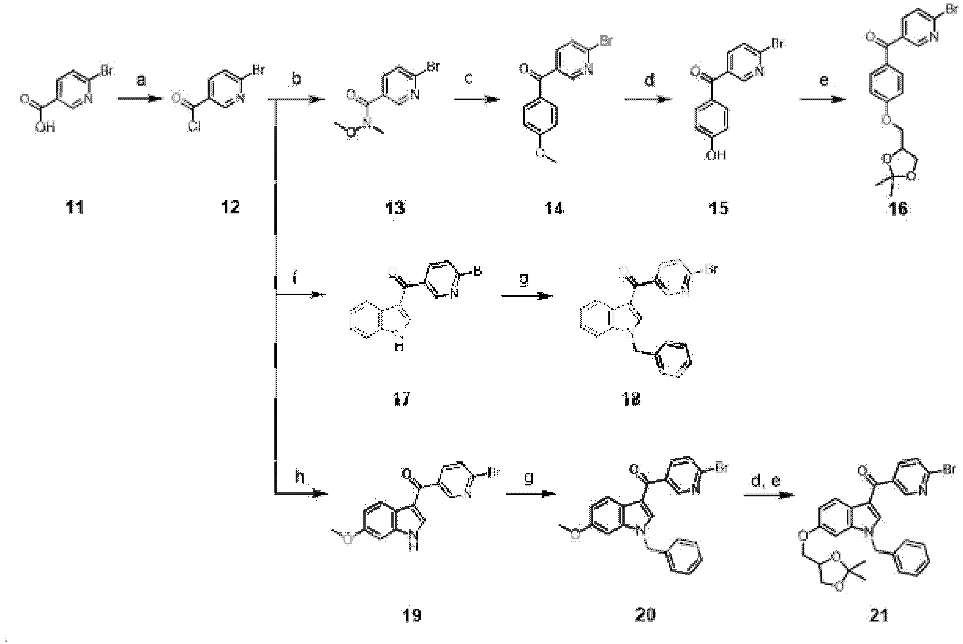

Reagents and conditions: (a) thionyl chloride, reflux; (b) N,O-dimethyl hydroxylamine, Et₃N, CH₂Cl₂, r.t., 62%; (c) 4-methoxyphenyl magnesium bromide, THF, 0°C, 87%; (d) BBr₃, CH₂Cl₂, -78°C to 0°C, 79-87%; (e) (2,2-dimethyl-1,3-dioxolan-4-yl)methyl 4-methylbenzene sulfonate, K₂CO₃, DMF, 70°C, 54-83%; (f) indole, AlCl₃, CH₂Cl₂, 0°C to r.t., 42%; (g) BnBr, NaH, DMF, 0°C, 95-97%; (h) 6-methoxyindole, AlCl₃, ZnCl₂, EtMgBr, CH₂Cl₂, r.t., 24%.

Compound 12, which is produced by refluxing commercially available 6-bromonicotinic acid (Compound 11) and thionyl chloride, was used for subsequent reactions without purification. After preparing Weinreb amide and introducing a methoxyphenyl group, Compound 15 was obtained through demethylation, and intermediate Compound 16 was obtained by introducing solketal ((6-bromopyridin-3-yl)(4-((2,2-dimethyl-1,3-dioxolan-4-yl)methoxy)phenyl)methanone); ¹H NMR (400 MHz, Chloroform-d) δ 8.73 (dd, *J* = 2.4, 0.8 Hz, 1H), 8.05 (dd, *J* = 8.2, 2.5 Hz, 1H), 7.48 (dd, *J* = 8.3, 0.9 Hz, 1H), 7.39 - 7.32 (m, 2H), 7.06 - 6.96 (m, 2H), 4.52 (p, *J* = 5.7 Hz, 1H), 4.28 (tt, *J* = 6.0, 5.1 Hz, 2H), 3.77 (dd, *J* = 8.7, 5.1 Hz, 2H), 1.48 (s, 3H), 1.42 (s, 3H).

By introducing indole into Compound 12, intermediate Compound 17 was synthesized ((6-bromopyridin-3-yl)(1H-indole-3-yl)metanone); ¹H NMR (400 MHz, DMSO-d₆) δ 12.24 (s, 1H), 8.79 (d, *J* = 3.1 Hz, 0H), 8.27 - 8.18 (m, 2H), 7.69 (s, 1H), 7.52 (s, 1H), 7.31 - 7.24 (m, 1H), 7.26 (s, 2H), and through benzylation thereof, intermediate Compound 18 was obtained ((1-benzyl-1H-indol-3-yl)(6-bromopyridin-3-yl)methanone); ¹H NMR (400 MHz, Chloroform-d) δ 8.81 (dd, *J* = 2.4, 0.8 Hz, 1H), 8.45 - 8.38 (m, 1H), 8.10 (dt, *J* = 8.4, 2.0 Hz, 1H), 7.60 (d, *J* = 1.5 Hz, 1H), 7.47 (dt, *J* = 8.2, 1.1 Hz, 1H), 7.36 (s, 2H), 7.42 - 7.29 (m, 5H), 7.26 (s, 1H), 7.21 - 7.12 (m, 2H), 5.38 (s, 2H).

The coupling reaction between 6-methoxyindole and Compound 12 was performed, a protecting group was introduced with a benzyl group, and intermediate Compound 21 was obtained by demethylation and introduction of solketal ((1-benzyl-6-(((2,2-dimethyl-1,3-dioxolan-4-yl)methoxy)-1H-indol-3-yl)(5-bromopyridin-2-yl)methanone); ¹H NMR (400 MHz, Chloroform-d) δ 8.80 (d, *J* = 2.4 Hz, 1H), 8.30 (d, *J* = 8.7 Hz, 1H), 8.12 - 8.05 (m, 1H), 7.53 - 7.42 (m, 2H), 7.34 (dd, *J* = 7.3, 3.2 Hz, 2H), 7.15 (t, *J* = 9.3 Hz, 3H), 7.02 (dd, *J* = 8.8, 2.2 Hz, 1H), 6.83 (d, *J* = 2.3 Hz, 1H), 5.31 (d, *J* = 4.2 Hz, 3H), 4.48 (q, *J* = 6.0 Hz, 1H), 4.17 (dd, *J* = 8.4, 6.4 Hz, 1H), 4.08 (dd, *J* = 9.4, 5.4 Hz, 1H), 3.93 (ddd, *J* = 19.6, 8.9, 5.7 Hz, 1H), 1.46 (s, 3H), 1.41 (s, 3H).

### [Example 1]

### Preparation of N-cyclopropyl-3-(5-(4-(2,3-dihydroxypropoxy)benzoyl)pyridin-2-yl)-5-fluoro-4-methylbenzamide (Compound 1)

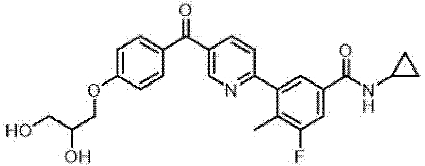

Compound 1 of the present disclosure was prepared in accordance with the following Preparation Scheme 1.

### [Preparation Scheme 1]

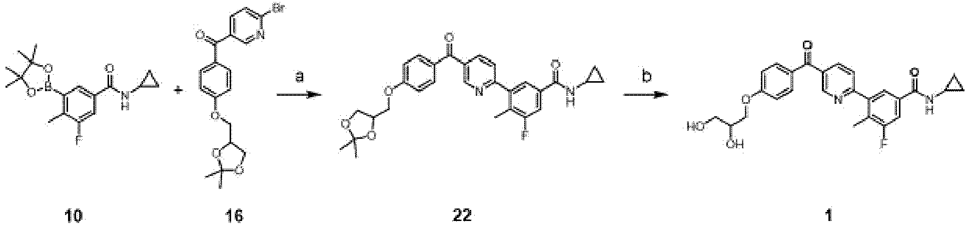

Reagents and conditions: (a) Pd(PPh₃)₄, K₂CO₃, DMF, 90°C, 36%; (b)p-TsOH, H₂O, MeOH, 50°C, 54%.

### 1-1. Synthesis of intermediate Compound 22

Intermediate Compound 10 (100.8 mg, 0.316 mmol) and intermediate Compound 16 (82.6 mg, 0.210 mmol), K₂CO₃ (58.0 mg, 0.420 mmol), and Pd(PPh₃)₄ (24.3 mg, 0.021 mmol) were dissolved in dry DMF, and argon substitution was performed, followed by stirring at 90°C for one hour. After checking that the reaction was terminated with TLC, the temperature of the reactant was lowered to room temperature, and the reaction was terminated using saturated aqueous NH₄Cl solution. The product was extracted into the organic layer with EtOAc and H₂O and then dehydrated using MgSO₄, and a filtered filtrate was distilled under reduced pressure and purified using flash column chromatography to synthesize intermediate Compound 22 (37.8 mg, 36%); ¹H NMR (400 MHz, Chloroform-d) δ 9.04 (dd, *J* = 2.3, 1.0 Hz, 1H), 8.17 (dd, *J* = 8.1, 2.2 Hz, 1H), 7.88 (dt, *J* = 9.6, 2.8 Hz, 2H), 7.61 (d, *J* = 1.9 Hz, 1H), 7.60 - 7.52 (m, 2H), 7.04 (dd, *J* = 9.2, 2.3 Hz, 2H), 6.40 (s, 1H), 4.52 (q, *J* = 4.6, 3.3 Hz, 1H), 4.25 - 4.10 (m, 2H), 4.10 - 4.02 (m, 1H), 3.98 - 3.90 (m, 1H), 2.94 - 2.87 (m, 1H), 2.35 (d, *J* = 2.4 Hz, 3H), 1.42 (m, 1H), 0.92 - 0.81 (m, 2H), 0.67 - 0.58 (m, 2H).

### 1-2. Synthesis of Compound 1

500 µL of H₂O and 2 mL of MeOH were added dropwise into intermediate Compound 22 (37.8 mg, 0.075 mmol) and p-TsOH (11.4 mg, 0.060 mmol), and stirring was performed at room temperature. After checking that the starting material disappeared with TLC, dilution was performed with CH₂Cl₂ and H₂O, and then the organic layer was extracted, followed by dehydration using Na₂SO₄. The filtered filtrate was distilled under reduced pressure and then purified using flash column chromatography to finally obtain Compound 1 (19 mg, 54%); ¹H NMR (500 MHz, DMSO-d₆) δ 8.92 (dd, *J* = 2.3, 0.9 Hz, 1H), 8.55 (d, *J* = 4.2 Hz, 1H), 8.17 (dd, *J* = 8.1, 2.2 Hz, 1H), 7.86 - 7.79 (m, 1H), 7.82 - 7.71 (m, 3H), 7.67 (dd, *J* = 10.4, 1.7 Hz, 1H), 7.16 - 7.05 (m, 2H), 5.02 (d, *J* = 5.2 Hz, 1H), 4.70 (t, *J* = 5.7 Hz, 1H), 4.16 - 4.04 (m, 1H), 4.03 - 3.93 (m, 1H), 3.85 - 3.75 (m, 1H), 3.50 - 3.40 (m, 2H), 2.88 - 2.77 (m, 1H), 2.28 (d, *J* = 2.3 Hz, 3H), 0.73 - 0.60 (m, 2H), 0.60 - 0.48 (m, 2H).

### [Example 2]

### Preparation of 6-chloro-N-cyclopropyl-4'-(1H-indole-3-carbonyl)-[1,1'-biphenyl]-3-carboxamide (Compound 2)

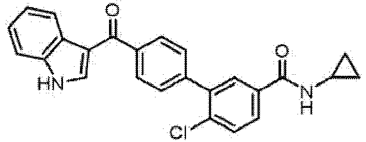

Compound 2 of the present disclosure was prepared according to the following Preparation Scheme 2.

### [Preparation Scheme 2]

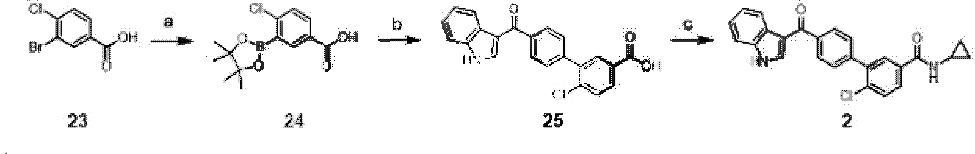

Reagents and conditions: (a) bis(pinacolato)diboron, PdCl₂(dppf)₂, KOAc, DMF, 90°C, 37%; (b) 17, PdCl₂(PPh₃)₂, K₃PO₄, 1,2-dioxane, H₂O, reflux, 10%; (c) cyclopropylamine, ethyl-3-(3-dimethylaminopropyl)carbodiimide), N,N-diisopropylethylamine,1-hydroxybenzotriazole hydrate, CH₂Cl₂, r.t., 12%.

### 2-1. Synthesis of intermediate Compound 24

Commercially available 3-bromo-4-chlorobenzoic acid (Compound 23, 1.5 g, 6.3 mmol), bis(pinacolato)diboron (2.6 g, 10.5 mmol), KOAc (3.1 g, 31.8 mmol), and PdCl₂(dppf)₂ were dissolved in 23 mL of dry DMF, and the reaction was carried out at 90°C after argon substitution. After checking that the reaction was terminated with TLC, the temperature of a reactant was lowered to room temperature. Saturated aqueous NH₄Cl solution was used to terminate the reaction, and the product was extracted into the organic layer with EtOAc and H₂O, followed by dehydration using MgSO₄. The filtered filtrate was distilled under reduced pressure and purified using flash column chromatography to obtain intermediate Compound 24 (664 mg, 37%).

### 2-2. Synthesis of intermediate Compound 25

Intermediate Compound 17 (500 mg, 1.67 mmol) and intermediate Compound 24 (659 mg, 2.33 mmol), PdCl₂(PPh₃)₂ (117 mg, 0.17 mmol), and K₃PO₄ (1.8 g, 8.33 mmol) were dissolved in a solvent mixed with 9 mL of 1,2-dioxane and 3 mL of H₂O, followed by reflux after argon substitution. Termination of the reaction was checked with TLC after 18 hours, and the temperature of the reactants was lowered to room temperature. Filtrates remaining after filtration with Celite were diluted with H₂O and CH₂Cl₂, resulting solids were filtered, and the filtrate was vacuum-depressurized to remove the remaining solvent and synthesize intermediate Compound 25 without any purification process (101 mg, 10%).

### 2-3. Synthesis of Compound 2

Intermediate Compound 25 (50 mg, 0.133 mmol) and 1-hydroxybenzotriazole hydrate (29 mg, 0.213 mmol) were dissolved in 0.7 mL of dry DMF, and 35 µL of N,N-diisopropylethylamine and 47 µL of ethyl-3-(3-dimethylaminopropyl) carbodiimide were added dropwise in order after argon substitution. After checking that the reaction was terminated with TLC, the reaction was terminated using H₂O, and the product was extracted into the organic layer using EtOAc and H₂O, followed by dehydration using MgSO₄. The filtered filtrate was distilled under reduced pressure and purified using flash column chromatography to finally obtain Compound 2 (6 mg, 12%); ¹H NMR (500 MHz, DMSO-d₆) δ 12.15 (d, *J* = 3.0 Hz, 1H), 8.63 (d, *J* = 4.2 Hz, 1H), 8.34 - 8.27 (m, 1H), 8.04 (d, *J* = 3.0 Hz, 1H), 7.97 - 7.91 (m, 3H), 7.90 (dd, *J* = 8.4, 2.2 Hz, 1H), 7.73 (d, *J* = 8.4 Hz, 1H), 7.71 - 7.64 (m, 2H), 7.59 - 7.53 (m, 1H), 7.34 - 7.24 (m, 2H), 2.93 - 2.84 (m, 1H), 0.76 - 0.66 (m, 2H), 0.66 - 0.56 (m, 2H).

### [Example 3]

### Preparation of N-cyclopropyl-3-(5-(6-(2,3-dihydroxypropoxy)-1H-indole-3-carbonyl)pyridin-2-yl)-5-fluoro-4-methylbenzamide (Compound 3)

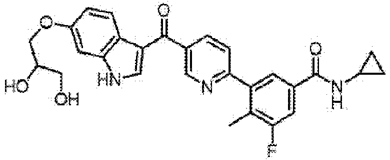

Compound 3 of the present disclosure was prepared according to the following Preparation Scheme 3.

### [Preparation Scheme 3]

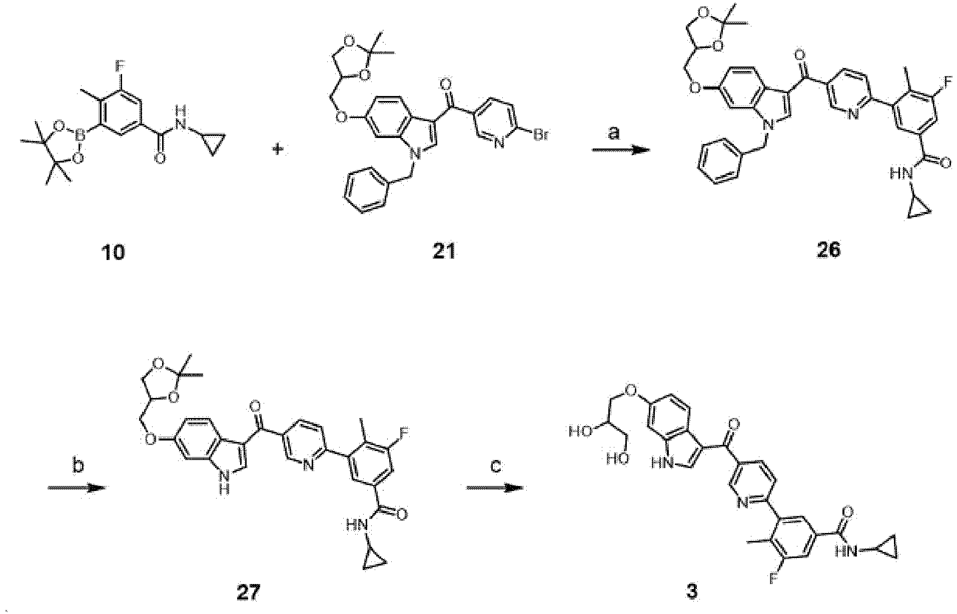

Reagents and conditions: (a) Pd(PPh₃)₄, K₂CO₃, DMF, 90°C, 100%; (b) potassium *tert*-butoxide, O₂, DMSO, 0°C, 12%; (c) *p*-TsOH, H₂O, MeOH, 50°C, 67%.

### 3-1. Synthesis of intermediate Compound 26

Using intermediate Compound 21 (127 mg, 0.244 mmol) as a stating material, intermediate Compound 26 was synthesized via the same method as that of intermediate Compound 22 (158 mg, 100%); ¹H NMR (400 MHz, Chloroform-d) δ 9.12 (s, 1H), 8.36 (d, *J* = 8.8 Hz, 1H), 8.22 (dd, *J* = 8.0, 2.2 Hz, 1H), 7.62 - 7.47 (m, 4H), 7.43 - 7.27 (m, 3H), 7.18 - 7.11 (m, 2H), 7.04 (dd, *J* = 8.8, 2.2 Hz, 1H), 6.84 (d, *J* = 2.2 Hz, 1H), 6.29 (s, 1H), 5.34 (s, 2H), 4.50 (p, *J* = 6.0 Hz, 1H), 4.18 (dd, *J* = 8.5, 6.4 Hz, 1H), 4.11 - 4.04 (m, 1H), 3.94 (ddd, *J* = 20.4, 8.9, 5.8 Hz, 2H), 2.91 (dt, *J* = 7.3, 3.8 Hz, 1H), 2.35 (d, *J* = 2.4 Hz, 3H), 2.28 - 2.22 (m, 1H), 2.05 (d, *J* = 0.8 Hz, 7H), 2.04 (s, 1H), 1.47 (s, 2H), 1.41 (s, 2H), 0.88 (q, *J* = 6.4 Hz, 3H), 0.62 (q, *J* = 6.0, 5.5 Hz, 2H).

### 3-2. Synthesis of intermediate Compound 27

O₂ was bubbled in a mixture in which intermediate Compound 26 (158 mg, 0.249 mmol) was dissolved in 0.8 mL of DMSO, and then potassium tert-butoxide (1.0 M in THF, 1.5 mL) was slowly added dropwise at 0°C. After checking that the reaction was terminated with TLC, dilution was performed with EtOAc, and the reaction was terminated using saturated aqueous NH₄Cl solution. After extracting the product into the organic layer with EtOAc and water, dehydration was performed using MgSO₄, the filtered filtrate was distilled under reduced pressure and then purified using flash column chromatography to synthesize intermediate Compound 27 (18.1 mg, 12%).

### 3-3. Synthesis of Compound 3

Using intermediate Compound 27 (10.0 mg, 0.018 mmol) as a stating material, Compound 3 was finally obtained via the same method as that of Compound 1 (6 mg, 67%); ¹H NMR (400 MHz, Methanol-d4) δ 9.05 (d, *J* = 3.7 Hz, 1H), 8.35 (dt, *J* = 7.9, 2.7 Hz, 1H), 8.21 (dd, *J* = 8.9, 3.3 Hz, 1H), 7.87 (dd, *J* = 3.5, 1.7 Hz, 1H), 7.82 - 7.72 (m, 2H), 7.67 (d, *J* = 10.2 Hz, 1H), 7.09 (t, *J* = 2.8 Hz, 1H), 7.06 - 6.98 (m, 1H), 4.17 - 4.11 (m, 1H), 4.10 - 4.02 (m, 2H), 3.77 - 3.70 (m, 2H), 2.88 (d, *J* = 7.0 Hz, 1H), 2.36 (q, *J* = 2.5 Hz, 3H), 2.03 (s, 1H), 1.31 (s, 1H), 0.83 (d, *J* = 6.6 Hz, 2H), 0.66 (d, *J* = 5.7 Hz, 2H).

### [Example 4]

### Preparation of 3-(5-(1H-indole-3-carbonyl)pyridin-2-yl)-N-cyclopropyl-5-fluoro-4-methylbenzamide (Compound 4)

.

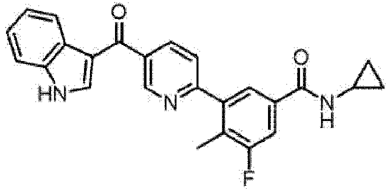

Compound 4 of the present disclosure was prepared according to the following Preparation Scheme 4.

### [Preparation Scheme 4]

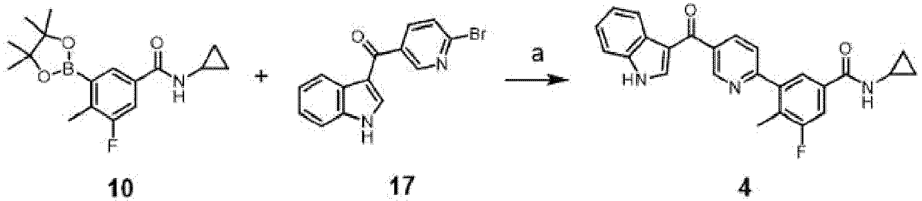

Reagents and conditions: (a) Pd(PPh₃)₄, K₂CO₃, dimethoxyethane, toluene, isopropyl alcohol, H₂O, MW, 84%.

In a reaction vessel containing intermediate Compound 10 (48 mg, 0.15 mmol), intermediate Compound 17 (30 mg, 0.1 mmol), Pd(PPh₃)₄ (11.6 mg, 0.01 mmol), and K₂CO₃ were dissolved in 2 mL of solvent (dimethoxyethane: toluene: isopropyl alcohol: H₂O = 10: 1: 3: 6), and, after argon substitution, the vessel was subjected to a reaction at 130°C for 30 minutes in a microwave reactor. After the mixture having the reaction terminated was cooled to room temperature, the reaction was terminated using water, and the product was extracted into the organic layer with EtOAc and H₂O and then dehydrated using MgSO₄. The filtrate filtered was distilled under reduced pressure and purified using flash column chromatography to finally obtain Compound 4 (34.8 mg, 84%); ¹H NMR (400 MHz, DMSO-d₆) δ 12.25 (s, 1H), 9.07 (s, 1H), 8.60 (d, *J* = 3.7 Hz, 1H), 8.31 (d, *J* = 7.5 Hz, 2H), 8.14 (s, 1H), 7.85 (s, 1H), 7.80 (d, *J* = 8.0 Hz, 1H), 7.72 (d, *J* = 10.9 Hz, 1H), 7.55 (d, *J* = 8.5 Hz, 1H), 7.34 - 7.25 (m, 2H), 2.87 (dq, *J* = 7.6, 4.2, 3.3 Hz, 1H), 2.34 (s, 3H), 0.75 - 0.67 (m, 2H), 0.61-0.57 (m, 2H).

### [Example 5]

### Preparation of 3-(5-(1-benzyl-1H-indole-3-carbonyl)pyridin-2-yl)-N-cyclopropyl-5-fluoro-4-methylbenzamide (Compound 5)

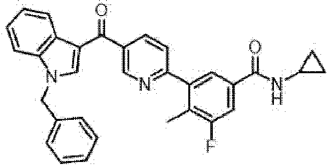

Compound 5 of the present disclosure was prepared in accordance with the Preparation Scheme 5.

### [Preparation Scheme 5]

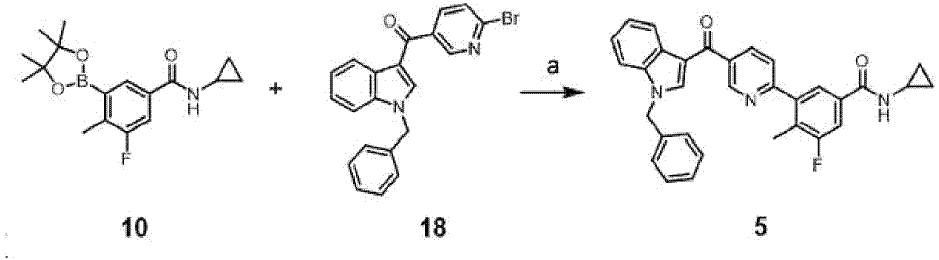

Reagents and conditions: (a) Pd(PPh₃)₄, K₂CO₃, DMF, 90°C, 94%.

Using intermediate Compound 18 (24.6 mg, 0.063 mmol) as a starting material, Compound 5 was finally obtained by the same method as that of intermediate Compound 22 (29.7 mg, 94%); ¹H NMR (400 MHz, Chloroform-d) δ 9.13 (dd, *J* = 2.2, 0.9 Hz, 1H), 8.48 (dd, *J* = 8.2, 1.7 Hz, 1H), 8.23 (dd, *J* = 8.0, 2.2 Hz, 1H), 7.69 (s, 1H), 7.62 - 7.52 (m, 3H), 7.43 - 7.25 (m, 6H), 7.17 (dd, *J* = 7.5, 2.0 Hz, 2H), 6.35 (s, 1H), 5.41 (s, 2H), 2.98 - 2.82 (m, 1H), 2.35 (d, *J* = 2.5 Hz, 3H), 0.96 - 0.81 (m, 2H), 0.67 - 0.55 (m, 2H).

### [Example 6]

### Preparation of N-cyclopropyl-3-(5-(1-(2,3-dihydroxypropyl)-1H-indole-3-carbonyl)pyridin-2-yl)-5-fluoro-4-methylbenzamide (Compound 6)

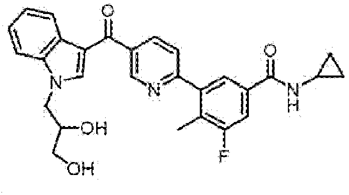

Compound 6 of the present disclosure was prepared in accordance with the following Preparation Scheme 6.

### [Preparation Scheme 6]

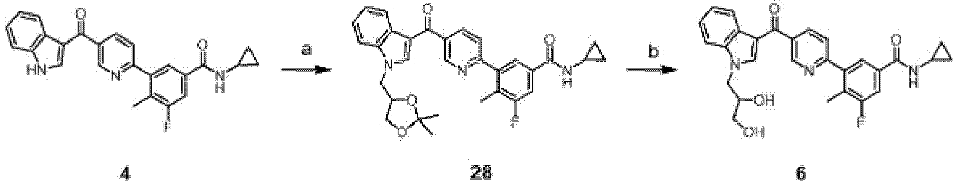

Reagents and conditions: (a) (2,2-dimethyl-1,3-dioxolan-4-yl)methyl 4-methylbenzene sulfonate, NaH, DMF, 90°C, 19%; (b) p-TsOH, H₂O, MeOH, 50°C, 62%.

### 6-1. Synthesis of intermediate Compound 28

After dissolving Compound 4 (50 mg, 0.121 mmol) in 1.5 mL of dry DMF and adding NAH (60% dispersion in mineral oil, 7.24 mg, 0.181 mmol) at 0°C, the mixture was stirred after argon substitution. After 10 minutes, (2,2-dimethyl-1,3-dioxolan-4-yl)methyl 4-methylbenzene sulfonate (52.0 mg, 0.181 mmol) and 4-dimethylaminopyridine (catalytic amount) were added, and a reaction was carried out by increasing the temperature slowly to 90°C. When the starting material disappeared after checking the TLC, the temperature of the mixture was cooled to room temperature, the reaction was terminated using saturated aqueous NH₄Cl solution, and the product was extracted into the organic layer with EtOAc and brine and then dehydrated using MgSO₄. The filtrate filtered was distilled under reduced pressure and purified using flash column chromatography to synthesize intermediate Compound 28 (13.5 mg, 19%).

### 6-2. Synthesis of Compound 6

Using intermediate Compound 28 (12 mg, 0.023 mmol) as a starting material, Compound 6 was finally obtained via the same method as that of Compound 1 (7 mg, 62%); ¹H NMR (500 MHz, Chloroform-d) δ 9.11 (s, 1H), 8.48 - 8.44 (m, 1H), 8.22 (dd, *J* = 8.1, 2.2 Hz, 1H), 7.76 (s, 1H), 7.59 (s, 1H), 7.56 (s, 1H), 7.54 (s, 1H), 7.48 - 7.43 (m, 1H), 7.38 (dd, *J* = 6.2, 3.1 Hz, 2H), 6.35 (s, 1H), 4.39 (dd, *J* = 14.5, 4.4 Hz, 1H), 4.26 (dd, *J* = 14.5, 7.3 Hz, 1H), 4.16 (s, 1H), 3.76 (d, *J* = 10.7 Hz, 1H), 3.58 (s, 1H), 2.89 (dd, *J* = 7.0, 3.5 Hz, 1H), 2.52 (s, 1H), 2.35 (d, *J* = 2.4 Hz, 3H), 1.40 (m, 1H), 0.87 (d, *J* = 6.0 Hz, 2H), 0.63 (d, *J* = 8.1 Hz, 1H).

### [Experimental Example 1]

### Evaluation of lipophilicity through Calculated LogP (CLogP)

Using Chemdraw software, structural formulas of Compounds 1 to 6 were filled in, and the CLogP values were checked, the results of which are shown in Table 2 below.

**TABLE 2**

| Compound Number | CLogP |
|---|---|
| 1 | 2.05 |
| 2 | 3.96 |
| 3 | 2.24 |
| 4 | 4.49 |
| 5 | 5.70 |
| 6 | 3.35 |

As shown in Table 2 above, according to Lipinski's rule of five, which was written by examining existing drugs, if the CLogP is less than 5, it has the characteristics of a drug that is easy to be administered orally. Therefore, it may be seen that the compound of the present disclosure may be developed as a drug for oral administration.

### [Experimental Example 2]

### Evaluation of p38α mitogen-activated protein kinase (MAPK) inhibitory ability

To determine the ability to inhibit p38 MAPK, Compounds 1 to 5 were diluted to determine the inhibitory effect depending on a concentration. Briefly, the compounds were diluted at concentrations of 1000, 333, 111, 37.0, 12.3, 4.12, 1.37, 0.457, 0.152, and 0.0495 nM, respectively, and then a peptide/kinase mixture (final 7.07-28.3 ng p38 alpha, 2 µM serine/threonine 15, 50 mM HEPES pH 7.0, 0.01% NaN3) and ATP solution were added respectively. After reacting at room temperature for 1 hour, a development reagent was added, followed by a reaction again at room temperature for 1 hour. Kinase inhibitory ability was identified at wavelengths of 445 nm and 520 nm, the results of which are shown in Table 3 below.

**TABLE 3**

| Compound | IC₅₀ (nM) |
|---|---|
| 1 | 22.3 |
| 2 | 34.1 |
| 3 | 34.3 |
| 4 | 18.8 |
| 5 | 193 |

As shown in Table 3 above, the compound of the present disclosure has been identified as a potent inhibitor that inhibits p38 alpha by 50% at concentrations of 200 nM or less.

### [Experimental Example 3]

### Evaluation of p38 MAPK protein inhibitory ability in cells

In order to identify inhibition of p-p38 MAPK, inhibition of p38 MAPK was identified after treating each compound to Raw264.7 cells which are obtained by converting macrophages that induces inflammation according to p-p38 MAPK activity into cancerous cells. Briefly, Raw264.7 cells were cultured in Dulbecco's modified Eagle Medium supplemented with 10% fetal bovine serum and penicillin/streptomycin (100 U/ml). Raw264.7 cells were treated with 1 µM of Compounds 1 to 5 according to the present disclosure in a culture dish respectively and cultured for 24 hours in a CO₂ incubator, and then treated with 100 ng/ml of LPS, followed by additional culture for 6 hours in a CO₂ incubator.

After cell culture was terminated according to the experimental conditions, cells were washed with cold phosphate buffered saline (PBS). The cells were then collected and centrifuged at 1200 rpm for 3 minutes. The pellet was lysed with 2X sample buffer (1M Tris-HCl pH 6.8, 50% glycerol, 10% SDS, 2-mercaptoethanol, 1% bromophenol blue) and boiled at 100°C for 10 minutes. The prepared protein samples were subjected to sodium dodecyl sulfate poly acrylamide gel electrophoresis and transferred to PVDF membrane (Millipore). For a specific binding reaction between the protein in the PVDF membrane and antibody, blocking was performed in a 5% bovine serum albumin (BSA, RMbio) solution. Subsequently, the BSA solution was discarded, and the membrane was washed with PBS-T (0.5% Tween 20 in PBS). The binding reaction of the protein in the membrane and antibody was performed overnight at 4°C and washed again with PBS-T. Finally, a secondary antibody (Cell signaling Technology) conjugated with horse radish peroxidase (HRP) was diluted in PBS-T and reacted at room temperature for 2 hours. Luminata Forte HRP substrate (Millipore) was used to determine the amount of protein in the membrane, the results of which are shown in Table 4 below.

**TABLE 4**

| Compound | p-p38 inhibition (%) |
|---|---|
| 1 | 11.3 |
| 2 | 69.0 |
| 3 | 39.7 |
| 4 | 68.0 |
| 5 | 52.8 |

As shown in Table 4 above, as a result of immunoblot, it was found that the compound of the present disclosure effectively inhibits p38 phosphorylation in cells at a concentration of 1 µM.

### [Experimental Example 4]

### Evaluation of cytokine mRNA expression inhibitory ability according to inflammatory response

In order to identify inhibition of p-p38 MAPK, inhibition of p38 MAPK was identified after treating each compound to Raw264.7 cells obtained by converting macrophages that induces inflammation according to p-p38 MAPK activity cancerous cells. Briefly, Raw264.7 cells were cultured in Dulbecco's modified Eagle Medium supplemented with 10% fetal bovine serum and penicillin/streptomycin (100 U/ml). Raw264.7 cells were treated with 1 µM of Compounds 1 to 5 according to the present disclosure in a culture dish respectively and cultured for 24 hours in a CO₂ incubator, and then treated with 100 ng/ml of LPS, followed by additional culture for 24 hours in a CO₂ incubator.

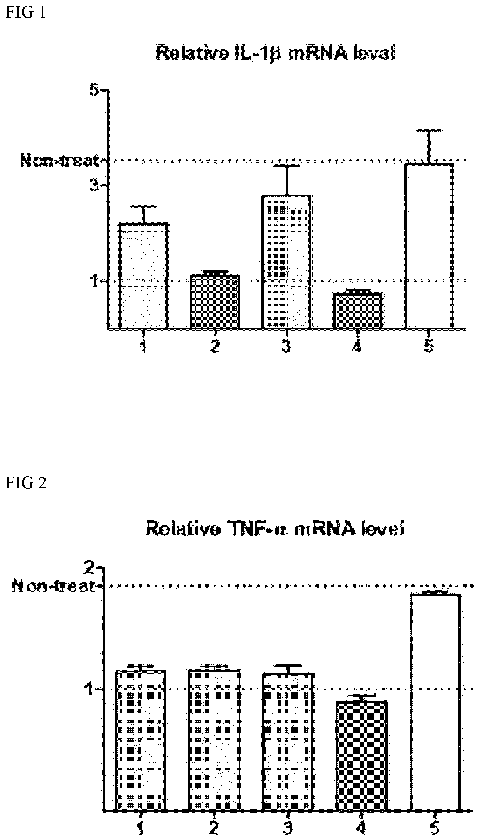

After cell culture was terminated according to the experimental conditions, cells were washed with cold phosphate buffered saline (PBS). The cells were then collected and centrifuged at 1200 rpm for 3 minutes. RNA was extracted according to the method provided in the Hybrid-R total RNA purification kit (GeneAll, 305-101). After measuring the concentration and purity of RNA using Nanodrop, cDNA was synthesized according to the method provided in TOPscript RT DryMIX (Enzynomics, RT200). After a reaction at 95°C for 3 minutes using SYBR Green Mix along with primer, cDNA was subjected to repetitive reactions 30 times at 95°C for 10 seconds, at 55°C for 10 seconds, and at 72°C for 30 seconds to determine the RNA expression level. The results are shown in Tables 5 and 6 below and FIGS. 1 and 2.

**TABLE 5**

| Compound | IL-1β mRNA inhibition (%) |
|---|---|
| 1 | 21.4 |
| 2 | 24.4 |
| 3 | 12.3 |
| 4 | 38.2 |
| 5 | 3.2 |

**TABLE 6**

| Compound | TNFα mRNA inhibition (%) |
|---|---|
| 1 | 38.1 |
| 2 | 37.6 |
| 3 | 39.2 |
| 4 | 51.5 |
| 5 | 4.1 |

As shown in Tables 5 and 6 and FIGS. 1 and 2, it was found that the compounds of the present disclosure inhibited mRNA expression of cytokines IL-1β and TNFα that generated an inflammatory response, and it may be seen that the compounds of the present disclosure inhibit production of inflammatory cytokines through inhibition of p38.

### [Experimental Example 5]

### Evaluation of TNFα inhibitory ability by cellular inflammatory response

In order to identify inhibition of p-p38 MAPK, inhibition of p38 MAPK was identified after treating each compound to Raw264.7 cells obtained by converting macrophages that induces inflammation according to p-p38 MAPK activity cancerous cells. Briefly, Raw264.7 cells were cultured in Dulbecco's modified Eagle Medium supplemented with 10% fetal bovine serum and penicillin/streptomycin (100 U/ml). Raw264.7 cells were treated with 1 µM of Compounds 1 to 5 according to the present disclosure in a culture dish respectively and cultured for 24 hours in a CO₂ incubator, and then treated with 100 ng/ml of LPS, followed by additional culture for 24 hours in a CO₂ incubator.

After the cell culture was completed according to the experimental conditions, the supernatant was obtained and centrifuged for 3 minutes at 1200 rpm. The centrifuged supernatant was dispensed into an antibody-coated plate, reacted at room temperature for 2 hours, and washed with PBS-T (0.5% tween 20 in PBS). After dispensing detection antibody including HRP, a reaction was performed at room temperature for 1 hour, and the amount of protein was checked at a wavelength of 450 nm, the results of which are shown in Table 7 below.

**TABLE 7**

| Compound | TNFα inhibition (%) |
|---|---|
| 1 | 22.1 |
| 2 | 68.5 |
| 3 | 16.7 |
| 4 | 76.6 |
| 5 | 23.8 |

As shown in Table 7, the compounds of the present disclosure inhibited generation of cytokine TNFα that generates the inflammatory response, and it may be found that the compounds of the present disclosure inhibit the inflammatory response through p38 inhibition.

### [Experimental Example 6]

### Evaluation of p38 MAPK protein inhibitory ability in brain-derived cells

In order to identify inhibition of p-p38 MAPK in brain cells, the inhibition of p38 MAPK was identified by inducing inflammation after treating each compound to SH-SY5Y cells obtained by converting human neurons into cancerous cells. In short, SH-SY5Y cells were cultured in Minimum essential medium/Eagle's balanced salts supplemented with 10% fetal bovine serum and penicillin/streptomycin (100 U/ml). SH-SY5Y cells were treated with 1 µM of Compounds 4 and 6 according to the present disclosure in a culture dish respectively and cultured for 24 hours in a CO₂ incubator, and then treated with 1000 ng/ml of LPS, followed by additional culture for 6 hours in a CO₂ incubator.

After cell culture was terminated according to the experimental conditions, cells were washed with cold phosphate buffered saline (PBS). The cells were then collected and centrifuged at 1200 rpm for 3 minutes. The cells in the sediment were lysed with 2X sample buffer (1M Tris-HCl pH 6.8, 50% glycerol, 10% SDS, 2-mercaptoethanol, 1% bromophenol blue) and boiled at 100°C for 10 minutes. The prepared protein samples were subjected to sodium dodecyl sulfate poly acrylamide gel electrophoresis and transferred to PVDF membrane (Millipore). For a specific binding reaction between the protein in the PVDF membrane and antibody, blocking was performed in a 5% bovine serum albumin (BSA, RMbio) solution. Subsequently, the BSA solution was discarded, and the membrane was washed with PBS-T (0.5% Tween 20 in PBS). The binding reaction of the protein in the membrane and antibody was performed overnight at 4°C and washed again with PBS-T. Finally, a secondary antibody (Cell signaling Technology) conjugated with horse radish peroxidase (HRP) was diluted in PBS-T and reacted at room temperature for 2 hours. Luminata Forte HRP substrate (Millipore) was used to determine the amount of protein in the membrane, the results of which are shown in Table 8 below.

**TABLE 8**

| Compound | p-p38 inhibition (%) |
|---|---|
| 4 | 74.6 |
| 6 | 22.8 |

As shown in Table 8 above, as a result of immunoblot, it was found that Compounds 4 and 6 of the present disclosure effectively inhibited p38 phosphorylation in cells derived from human at a concentration of 1 µM.

### [Experimental Example 7]

### Evaluation of p38 MAPK protein inhibitory ability in normal brain cells

In order to identify inhibition of p-p38 MAPK in normal cells rather than cancer cells, the inhibition of p38 MAPK was identified by inducing inflammation after treating neuron cells obtained from cerebrum of ICR mice with each compound. In short, neurons were collected from the cerebrum of the embryos of ICR mice 17-18 days after gestation and cultured for 7 days in a neurobasal medium supplemented with 2% B27 supplements, 2 mM glutamine, and penicillin/streptomycin (100 U/ml). Primary neurons were treated with 1 µM of Compounds 4 and 6 according to the present disclosure in a culture dish respectively and cultured for 24 hours in a CO₂ incubator, and then treated with 1000 ng/ml of LPS, followed by additional culture for 6 hours in a CO₂ incubator.

After the cell culture was terminated according to the experimental conditions, washing was performed with cold phosphate buffered saline (PBS). The cells were then collected and centrifuged at 1200 rpm for 3 minutes. The cells in the sediment were lysed with 2X sample buffer (1M Tris-HCl pH 6.8, 50 % glycerol, 10 % SDS, 2-mercaptoethanol, 1 % bromophenol blue) and boiled at 100°C for 10 minutes. The prepared protein samples were subjected to sodium dodecyl sulfate poly acrylamide gel electrophoresis and transferred to PVDF membrane (Millipore). For a specific binding reaction between the protein in the PVDF membrane and antibody, blocking was performed in a 5% bovine serum albumin (BSA, RMbio) solution. Subsequently, the BSA solution was discarded, and the membrane was washed with PBS-T (0.5% Tween 20 in PBS). The binding reaction between the protein in the membrane and antibody was performed overnight at 4°C and washed again with PBS-T. Finally, a secondary antibody (Cell signaling Technology) conjugated with horse radish peroxidase (HRP) was diluted in PBS-T and reacted at room temperature for 2 hours. Luminata Forte HRP substrate (Millipore) was used to determine the amount of protein in the membrane, the results of which are shown in Table 9 below.

**TABLE 9**

| Compound | p-p38 inhibition (%) |
|---|---|
| 4 | 55.8 |
| 6 | 60.7 |

As shown in Table 9 above, as a result of immunoblot, it was found that Compounds 4 and 6 of the present disclosure effectively inhibited p38 phosphorylation due to inflammation even in normal cells other than cancer cells at a concentration of 1 µM.

### [Experimental Example 8]

### Evaluation of cytokine mRNA expression inhibitory ability by inflammatory response

In order to identify inhibition of p-p38 MAPK, the inhibition of p38 MAPK was identified by treating each compound to BV2 cells obtained by converting microglia that induces inflammation according to p-p38 MAPK activity in the brain into cancerous cells. Briefly, BV2 cells were cultured in Dulbecco's modified Eagle Medium supplemented with 10% fetal bovine serum and penicillin/streptomycin (100 U/ml). BV2 cells were treated with 1 µM of Compounds 4 to 6 according to the present disclosure in a culture dish respectively and cultured for 24 hours in a CO₂ incubator, and then treated with 1000 ng/ml of LPS, followed by additional culture for 24 hours in a CO₂ incubator.

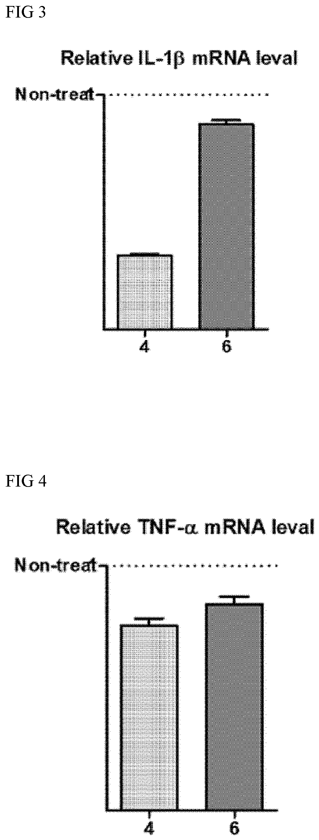

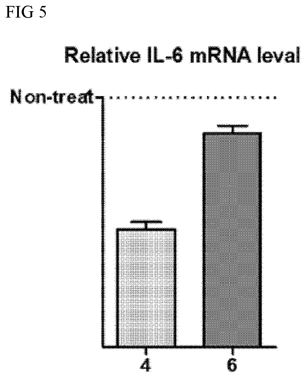

After the cell culture was completed according to the experimental conditions, washing was performed with cold phosphate buffered saline (PBS). The cells were then collected and centrifuged at 1200 rpm for 3 minutes. RNA was extracted according to the method provided in the Hybrid-R total RNA purification kit (GeneAll^{®}, 305-101). After measuring the concentration and purity of RNA using Nanodrop, cDNA was synthesized according to the method provided in TOPscript RT DryMIX (Enzynomics, RT200). After a reaction at 95°C for 3 minutes using SYBR Green Mix along with primer, cDNA was subjected to repetitive reactions 30 times at 95°C for 10 seconds, at 55°C for 10 seconds, and at 72°C for 30 seconds to determine the RNA expression level. The results are shown in Tables 10 to 12 and FIGS. 3 to 5 below.

**TABLE 10**

| Compound | IL-1β mRNA inhibition (%) |
|---|---|
| 4 | 68.5 |
| 6 | 12.6 |

**TABLE 11**

| Compound | TNFα mRNA inhibition (%) |
|---|---|
| 4 | 24.5 |
| 6 | 15.7 |

**TABLE 12**

| Compound Number | IL-6 mRNA inhibition (%) |
|---|---|
| 4 | 52.8 |
| 6 | 14.4 |

As shown in Tables 10 to 12 and FIGS. 3 to 5, the compounds of the present disclosure inhibited mRNA generation for inflammatory cytokines IL-1β, TNFα and IL-6, and it may be seen that the compounds of the present disclosure inhibit expression of inflammatory cytokines through p38 inhibition.

## Claims

1. A compound represented by the following Chemical Formula 1 or a pharmaceutically acceptable salt thereof; 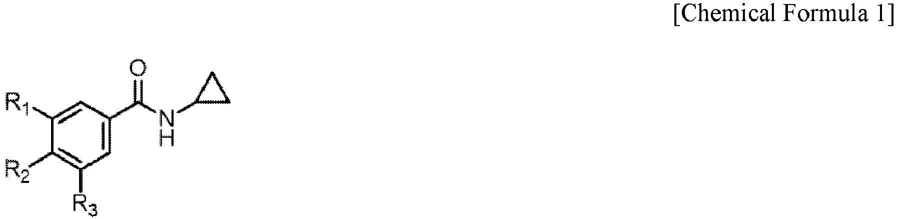 wherein, in the Chemical Formula,
R₁ is 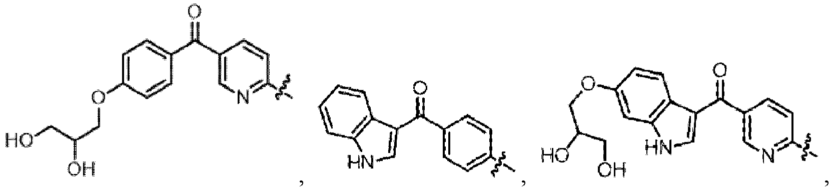 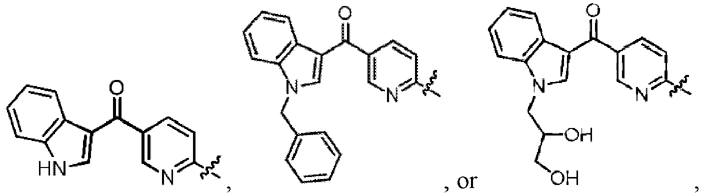
R₂ is H, halogen, or -C₁-C₆ alkyl, and
R₃ is H or halogen.

2. The compound or pharmaceutically acceptable salt thereof of claim 1, wherein the compound is selected from the group consisting of the following compounds:
| | |
|---|---|
| 1 | 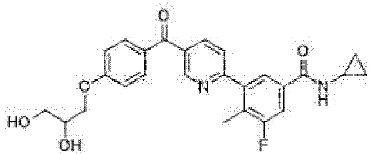 |
| 2 | 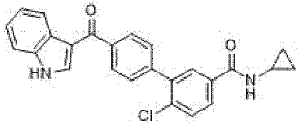 |
| 3 | 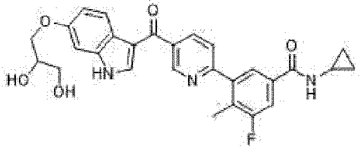 |
| 4 | 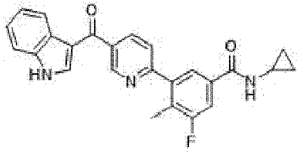 |
| 5 | 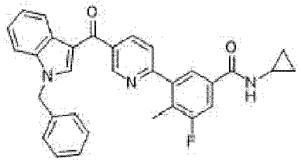 |
| 6 | 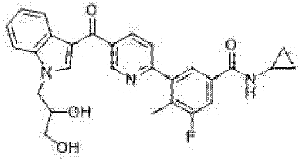 |

3. A pharmaceutical composition for treating or preventing an inflammatory disease, comprising the compound according to claim 1.

4. A pharmaceutical composition for treating or preventing an inflammatory disease, comprising the compound according to claim 2.
